# EUROPEAN PATENT APPLICATION

(11) **EP 4 166 080 A1**
(43) Date of publication of application: **19.04.2023**
(21) Application number: 21849733.7
(22) Date of filing: 24.06.2021
(51) Int. Cl.: A61B 5/16, A61B 5/0285, F24F 11/63

(54) **COMFORT DETERMINATION DEVICE, AIR CONDITIONER, AND COMFORT DETERMINATION METHOD**

(30) Priority: 30.07.2020 JP 2020129411
(71) Applicant: Daikin Industries, Ltd., Osaka-shi, Osaka 530-8323 (JP)
(72) Inventor: EMOTO, Shiori, Osaka-shi, Osaka 530-8323 (JP); NISHINO, Atsushi, Osaka-shi, Osaka 530-8323 (JP)
(74) Representative: Hoffmann Eitle
(86) International application number: PCT/JP2021/024041
(87) International publication number: WO 2022/024617

(57) **Abstract**

A comfort determination device (10) includes: an acquisition unit (11A) configured to acquire time series data on a blood flow of a target; a calculator (14A) configured to calculate an index of fluctuation indicating fluctuations in the blood flow of the target from the time series data on the blood flow acquired by the acquisition unit (11A); and a determiner (14B) configured to determine whether or not the target is comfortable, based on the index of fluctuation calculated by the calculator (14A).

## Description

### TECHNICAL FIELD

The present disclosure relates to a comfort determination device, an air conditioner, and a comfort determination method.

### BACKGROUND ART

A thermal sensation determination device of Patent Document 1 includes a skin temperature detector, a fluctuating state determiner, a gradient detector, and a thermal sensation determiner. The skin temperature detector detects the skin temperature of a peripheral portion of the body of a subject. The fluctuating state determiner determines the fluctuating state of the skin temperature over a first phase, and classifies the fluctuating state as any one of a first state where fluctuations in the skin temperature are the gentlest, a second state where the fluctuations are the intensest, and a third state other than the first and second states, thereby to obtain a fluctuating state determination result. The gradient detector detects the gradient of the skin temperature over a second phase, and classifies the gradient as any one of a first gradient that is the greatest, a second gradient that is smaller than the first gradient, and a third gradient that is smaller than the second gradient, thereby to acquire a gradient detection result. The thermal sensation determiner determines the subject's thermal sensation, using the fluctuating state determination result, thereby obtaining a thermal sensation determination result as one of levels between "hot" and "cold" with reference to "moderate."

The thermal sensation determination device of Patent Document 1 is based on the correlation between variations in the subject's skin temperature and the subject's thermal sensation, and is configured to output the subject's thermal sensation determination result.

### CITATION LIST

### PATENT DOCUMENT

Patent Document 1: Japanese Unexamined Patent Publication No. 2008-241135

### SUMMARY OF THE INVENTION

### TECHNICAL PROBLEM

However, the subject's skin temperature would possibly vary due to a factor different from the subject's thermal sensation. For example, sweating of the subject may lower the subject's skin temperature irrespective of the subject's thermal sensation. In addition, exposing the subject to wind may lower the subject's skin temperature irrespective of the subject's thermal sensation. The consequent loss of the correlation between variations in the subject's (target's) skin temperature and the subject's thermal sensation would deteriorate the accuracy of the thermal sensation determination result of determination as to whether the subject is comfortable ("moderate") or whether the subject is not comfortable ("hot" or "cold").

It is an object of the present disclosure to reduce accuracy deterioration of a determination result of determining whether or not a target is comfortable.

### SOLUTION TO THE PROBLEM

A first aspect of the present disclosure is directed to a comfort determination device (10). The comfort determination device (10) includes: an acquisition unit (11A) configured to acquire time series data on a blood flow of a target; a calculator (14A) configured to calculate an index of fluctuation indicating fluctuations in the blood flow of the target from the time series data on the blood flow acquired by the acquisition unit (11A); and a determiner (14B) configured to determine whether or not the target is comfortable, based on the index of fluctuation calculated by the calculator (14A).

The first aspect can reduce accuracy deterioration of the determination result of determination as to whether or not the target is comfortable.

A second aspect of the present disclosure is directed to the comfort determination device (10) according to the first aspect wherein the calculator (14A) calculates a variance of the time series data on the blood flow acquired by the acquisition unit (11A) as the index of fluctuation.

According to the second aspect, a determination is made as to whether or not the target is comfortable, based on the variance value of the time series data on the blood flow.

A third aspect of the present disclosure is directed to the comfort determination device (10) according to the second aspect wherein the determiner (14B) selects a threshold for determining whether or not the target is comfortable based on a maximum one of such variances of the target thus calculated in the past.

According to the third aspect, the threshold for determining whether or not the target is comfortable can be selected.

A fourth aspect of the present disclosure is directed to the comfort determination device (10) according to the third aspect, further including: a first input unit (12) configured to receive input of information indicating whether or not the target has felt comfortable; and a controller (14C) configured to select an appropriate coefficient based on a calculation result of the control device (14) and an input result received by the first input unit (12), the threshold indicating a value obtainable by multiplying the maximum one of the variances by the appropriate coefficient.

According to the fourth aspect, the appropriate coefficient can be selected based on the index of fluctuation in the target's blood flow calculated by the calculator (14A) and the information entered through the first input unit (12) and indicating whether or not the target is comfortable.

A fifth aspect of the present disclosure is directed to the comfort determination device according to the third aspect, further including: a second input unit (12) configured to receive input of information related to an attribute of the target; and a controller (14C) configured to select an appropriate coefficient based on an input result entered into the second input unit (12), the threshold indicating a value obtainable by multiplying the maximum variance by the appropriate coefficient.

According to the fifth aspect, the appropriate coefficient can be selected based on the target's attribute.

A sixth aspect of the present disclosure is directed to the comfort determination device according to any one of the third to fifth aspects, wherein the maximum one of the variances indicates a maximum value of the plurality of variances calculated within a predetermined period.

According to the sixth aspect, the plurality of variances of the blood flow are calculated within the predetermined period, and the calculated variances are compared to each other in order to determine the maximum variance among them.

A seventh aspect of the present disclosure is directed to the comfort determination device according to any one of the third to fifth aspects, wherein the maximum one of the variances indicates a maximum value of the plurality of variances calculated by the calculator (14A) while the target is determined as being at rest based on measurement results of a body motion sensor (40) measuring a state of the target.

According to the seventh aspect, a maximum value of the blood flow can be acquired based on the measurement result of the body motion sensor (40).

An eighth aspect of the present disclosure is directed to an air conditioner (30). The air conditioner (30) includes a comfort determination device (10) described above.

According to the eighth aspect, the air conditioner (30) can operate based on the determination result of the determiner (14B).

A ninth aspect of the present disclosure is directed to a comfort determination method. The comfort determination method includes: acquiring time series data on a blood flow of a target; calculating an index of fluctuation indicating fluctuations in the blood flow of the target from the time series data on the blood flow; and determining whether or not the target is comfortable, based on the index of fluctuation.

The ninth aspect can reduce accuracy deterioration of the determination result of the determination as to whether or not the target is comfortable.

### BRIEF DESCRIPTION OF THE DRAWINGS

FIG. 1 is a block diagram showing the configuration of a determination system according to an embodiment of the present invention.
FIG. 2 is a schematic diagram showing an operation of the determination system.
FIG. 3 is a flowchart showing an operation of a comfort determination device.
FIG. 4 is a schematic diagram showing a first example of a procedure of selecting an appropriate coefficient.
FIG. 5 is a block diagram showing the configuration of a determination system for selecting a maximum variance.
FIG. 6 is a flowchart showing a second example of a procedure of selecting the maximum variance.
FIG. 7 is a block diagram showing a configuration of a variation of the determination system.

### DESCRIPTION OF EMBODIMENTS

Embodiments of the present invention will be described in detail with reference to the drawings. Note that like reference characters denote the same or equivalent components in the drawings, and the detailed description thereof, the description of advantages associated therewith, and other descriptions will not be repeated.

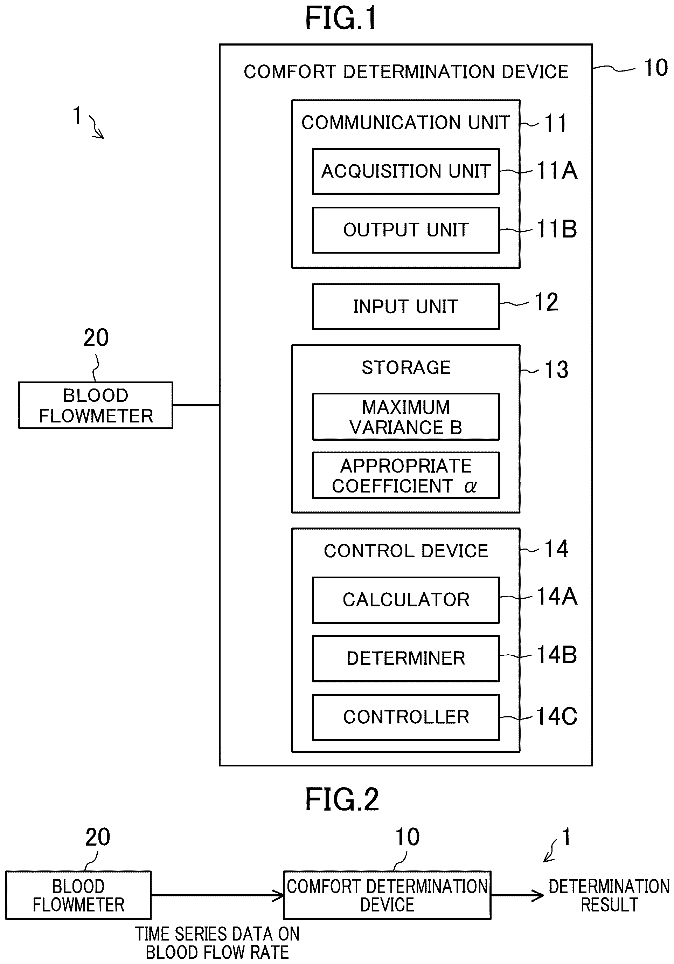

A determination system (1) according to an embodiment of the present invention will be described with reference to FIGS. 1 and 2. FIG. 1 is a block diagram showing the configuration of the determination system (1). FIG. 2 is a schematic diagram showing an operation of the determination system (1).

### -General Configuration-

As shown in FIGS. 1 and 2, the determination system (1) includes a comfort determination device (10) and a blood flowmeter (20).

The blood flowmeter (20) is configured to be fitted to a target to measure the target's blood flow. The blood flowmeter (20) is, for example, a laser Doppler blood-flowmeter. The blood flowmeter (20) is communicably connected to the comfort determination device (10) in a wired or wireless manner. Time series data on the target's blood flow measured by the blood flowmeter (20) are output to the comfort determination device (10). The time series data on the blood flow indicate plural pieces of data on the blood flow measured within a predetermined measurement period. In this embodiment, the time series data on the blood flow are obtained by successively measuring the blood flow on a time series basis (e.g., at intervals of several seconds).

The comfort determination device (10) determines whether or not the target is comfortable. In this embodiment, the comfort determination device (10) is a terminal such as a smartphone or a personal computer (PC). In this embodiment, the comfort determination device (10) includes an application program installed thereon to perform a determination process for determining whether or not the target is comfortable, and executes the application program to perform the determination process.

As illustrated in FIGS. 1 and 2, the comfort determination device (10) includes a communication unit (11), an input unit (12), a storage (13), and a control device (14).

The communication unit (11) is a device for communicating with an external device. The communication unit (11) is communicably connected to the blood flowmeter (20) in a wired or wireless manner. The communication unit (11) includes, for example, at least one of a device for performing wireless communication (such as a wireless LAN module) or a device for performing wired communication (such as a communication port connected to a communication cable). The communication unit (11) includes an acquisition unit (11A) configured to acquire information, and an output unit (11B) configured to output information. The acquisition unit (11A) acquires time series data on the target's blood flow output from the blood flowmeter (20).

The input unit (12) receives an external instruction for instructing the comfort determination device (10). The input unit (12) includes, for example, at least one of a touch panel, a keyboard, or an operation button.

The storage (13) includes a main memory (e.g., a semiconductor memory), such as a flash memory, a read only memory (ROM), and a random access memory (RAM), and may further include an auxiliary memory (e.g., a hard disk drive, a solid state drive (SSD), a secure digital (SD) memory card, or a universal serial bus (USB) flash memory). The storage (13) stores various computer programs executable by the control device (14).

The storage (13) stores therein a maximum variance B and an appropriate coefficient α.

The maximum variance B indicates a maximum one of the plurality of variances of the time series data on the blood flow of the target that have been calculated in the past.

The appropriate coefficient α is a coefficient for use in selecting a threshold for determining whether or not the target is comfortable. In this embodiment, the threshold is a value obtainable by multiplying the maximum variance B by the appropriate coefficient α (threshold = α·B). The appropriate coefficient α is a real number greater than zero but not greater than one (0 < α ≤ 1).

The control device (14) includes a processor, such as a central processing unit (CPU) or a microprocessor unit (MPU). The control device (14) executes the computer programs stored in the storage (13) to control elements of the comfort determination device (10). The control device (14) includes a calculator (14A), a determiner (14B), and a controller (14C). The control device (14) executes the computer programs stored in the storage (13) to function as the calculator (14A), the determiner (14B), and the controller (14C).

### -Outline of Operation of Determination System (1)-

The outline of operation of the determination system (1) will be described with reference to FIGS. 1 and 2.

As illustrated in FIGS. 1 and 2, the blood flowmeter (20) outputs the time series data on the target's blood flow to the comfort determination device (10). The comfort determination device (10) is so configured that the acquisition unit (11A) acquires the time series data on the blood flow from the blood flowmeter (20), and, from the time series data on the blood flow, the calculator (14A) calculates out an index of fluctuation indicating fluctuations in the target's blood flow. The determiner (14B) of the comfort determination device (10) determines whether or not the target is comfortable, based on the index of fluctuation calculated by the calculator (14A).

The index of fluctuation is the numerically expressed magnitude of fluctuations in the blood flow.

The state where the target is comfortable means that the target perceives that the air temperature in the environment where the target is present is not too high and not too low.

The state where the target is not comfortable (i.e., uncomfortable) means that the target perceives that the air temperature in the environment where the target is present is too high or too low.

A description will be given of the principle of why fluctuations in the target's blood flow are employed as an element used by the comfort determination device (10) to determine whether or not the target is comfortable.

The inventors of this application have considered, based on the human body mechanism that changes the blood flow in accordance with the air temperature to regulate the body temperature, that there is a correlation between the human sensitivity to the air temperature and fluctuations in the blood flow as described below.

When a human perceives the air temperature is too high, the blood vessels of the human dilate to lower the human body temperature. At this time, the diameter of the human blood vessels converges to a substantially uniform diameter to keep the human blood vessels dilated. This reduces fluctuations in the blood flow.

When a human perceives the air temperature is too low, the blood vessels of the human contract to raise the human body temperature. At this time, the diameter of the human blood vessels converges to a substantially uniform diameter to keep the human blood vessels contracted. This reduces fluctuations in the blood flow.

In contrast, when a human perceives the air temperature is appropriate, the body temperature regulation is not necessary. Thus, the blood vessel diameter regulation to converge to a substantially uniform diameter does not take place. This increases fluctuations in the blood flow.

From the foregoing consideration, the inventors of this application have found that observing fluctuations in the target's blood flow allows an objective determination as to whether or not the target is comfortable. As a result, the inventors of this application have employed fluctuations in the target's blood flow as an element used to determine whether or not the target is comfortable.

In addition, the inventors of this application have devised an index of fluctuation that is the numerically expressed magnitude of fluctuations in the blood flow so that the result of determining whether or not the target is comfortable can be calculated by arithmetic processing.

The index of fluctuation may be, for example, a variance of the time series data on the blood flow, the difference between the maximum and minimum of the time series data on the blood flow, the power spectrum of the time series data on the blood flow, the amplitude of the time series data on the blood flow, the period of the time series data on the blood flow, or the mean square error of the time series data on the blood flow. In this embodiment, the variance of the time series data on the blood flow is used as the index of fluctuation.

### -Operation of Comfort Determination Device (10)-

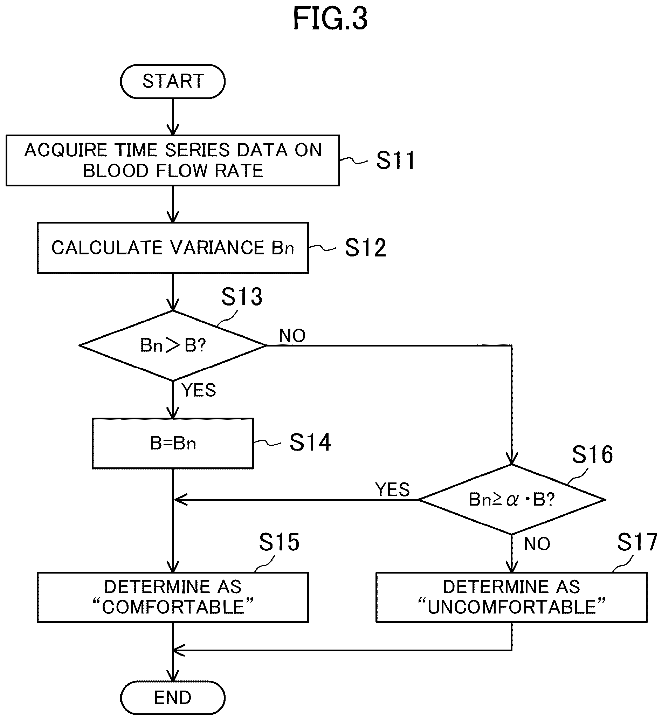

Next, an operation of the comfort determination device (10) will be described with reference to FIGS. 1 and 3. FIG. 3 is a flowchart showing an operation of the comfort determination device (10).

As illustrated in FIGS. 1 and 3, in step S11, the blood flowmeter (20) is fitted to the target to measure the target's blood flow. Then, the blood flowmeter (20) outputs the time series data on the target's blood flow to the comfort determination device (10). As a result, the acquisition unit (11A) of the comfort determination device (10) acquires the time series data on the blood flow.

In step S12, the calculator (14A) calculates out a variance Bn of the blood flow from the time series data on the blood flow acquired by the acquisition unit (11A).

In step S13, the determiner (14B) determines whether or not the variance Bn is greater than the maximum variance B (see FIG. 1) (Bn > B). If the determiner (14B) determines that the variance Bn is greater than the maximum variance B ("Yes" in step S13), the process proceeds to step S14. If the determiner (14B) determines that the variance Bn is not greater than the maximum variance B ("No" in step S13), the process proceeds to step S16.

In step S14, the determiner (14B) updates the maximum variance B stored in the storage (13) to the maximum variance Bn.

In step S15, the determiner (14B) determines that the target is comfortable. As a result, the process ends.

In step S16, the determiner (14B) determines whether or not the variance Bn is greater than or equal to the threshold α·B. If the determiner (14B) determines that the variance Bn is greater than or equal to the threshold α·B (Bn ≥ α·B) ("Yes" in step S16), the process proceeds to step S15. If the determiner (14B) determines that the variance Bn is neither greater than nor equal to the threshold α·B (Bn < α·B) ("No" in step S16), the process proceeds to step S17.

In step S17, the determiner (14B) determines that the target is uncomfortable. As a result, the process ends.

### -First Example of Procedure of Selecting Appropriate Coefficient α-

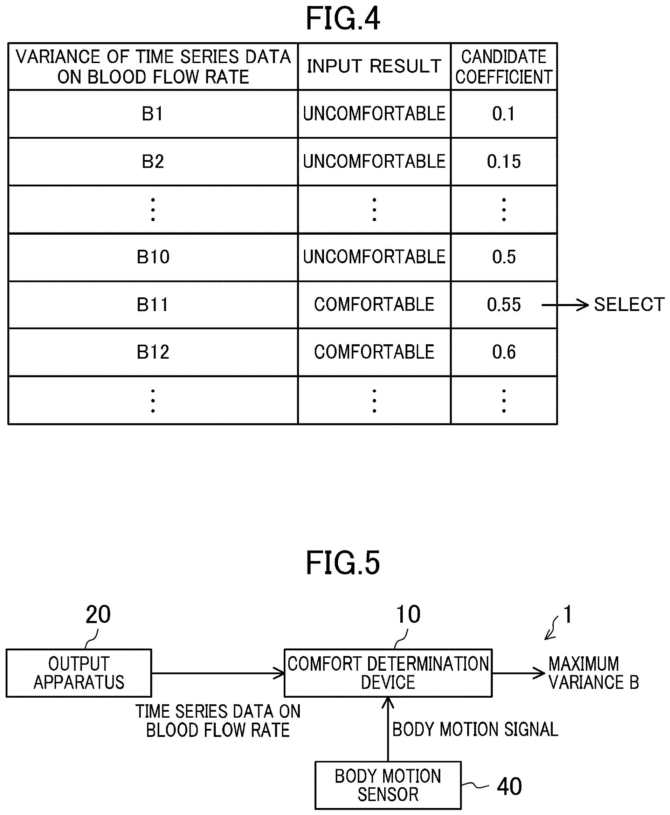

A first example of a procedure of selecting the appropriate coefficient α (see FIG. 1) will be described with reference to FIGS. 1 and 4. FIG. 4 is a schematic diagram showing the first example of the procedure of selecting the appropriate coefficient α.

As illustrated in FIGS. 1 and 4, in performing a process for selecting the appropriate coefficient α, the air temperature in the environment where the target is present is appropriately changed with the blood flowmeter (20) fitted to the target. At this time, every time the air temperature is changed, the blood flowmeter (20) measures the time series data on the target's blood flow, and outputs the measurement result to the comfort determination device (10). The controller (14C) of the comfort determination device (10) calculates out a plurality of variances Bx (x = 1, 2, 3, 4, ...) from the time series data on the blood flow acquired from the blood flowmeter (20). The plurality of variances Bx is associated respectively with the air temperatures thus changed as appropriate. For example, the variance B1 indicates the variance calculated from the time series data on the blood flow measured by the blood flowmeter (20) when the air temperature is changed to 16°C, and the variance B2 indicates the variance calculated from the time series data on the blood flow measured by the blood flowmeter (20) when the air temperature is changed to 17°C.

In such a case that the blood flowmeter (20) measures the time series data on the target's blood flow every time the air temperature is changed, the target operates the input unit (12) to enter information indicating whether or not the target has felt comfortable ("comfortable" or "uncomfortable"), every time the air temperature is changed.

As a result, a list in which the variances Bx are associated with the respective results ("comfortable" or "uncomfortable") entered by the target through the input unit (12) is created (see FIG. 4).

In the list shown in FIG. 4, the variance Bx of the time series data on the blood flow increases in the order of B1, B2, B3, B4, ... (B1 < B2 < B3 < B4 ...).

In the list shown in FIG. 4, candidate coefficients are values that are candidates for the appropriate coefficient α. In the first example, each candidate coefficient is a value obtainable by dividing the variance Bx by the maximum variance B (see FIG. 1) stored in the storage (13) (candidate coefficient = Bx/B).

The controller (14C) of the comfort determination device (10) selects, as the appropriate coefficient α, a minimum one of a plurality of candidate coefficients associated with the entered result "comfortable" in the list shown in FIG. 4. In this embodiment, the controller (14C) selects the candidate coefficient "0.55" as the appropriate coefficient α. Then, the controller (14C) causes the storage (13) to store the appropriate coefficient α therein.

### -Second Example of Procedure of Selecting Appropriate Coefficient α-

A second example of the procedure of selecting the appropriate coefficient α will be described.

As shown in FIG. 1, the storage (13) of the comfort determination device (10) stores a table (not shown) in which attributes are associated with the respective candidate coefficients. Examples of the types of the attributes include attributes based on gender (male or female), attributes based on physique (e.g., slender, normal, and large), attributes based on age (e.g., young and old), and attributes based on physical constitution (e.g., sensitive to the heat or the cold). In the table, for example, the "male" is associated with the candidate coefficient "0.8," and the "female" is associated with the candidate coefficient "0.75."

For a process for selecting the appropriate coefficient α, the input unit (12) receives input of information related to the target's attribute. That is to say, the target enters information related to his/her own attribute through the input unit (12). Then, the controller (14C) selects the appropriate coefficient α based on the result entered through the input unit (12). More specifically, the controller (14C) selects the candidate coefficient associated with the attribute entered through the input unit (12) in the table, as the appropriate coefficient α. For example, if information indicating "male" is entered through the input unit (12), the controller (14C) selects the candidate coefficient "0.8" associated with the "male" as the appropriate coefficient α.

The table may be such that each of the candidate coefficients is associated with a plurality of attributes. For example, the candidate coefficient "0.78" is associated with a plurality of attributes including "male," "slender," "young," and "sensitive to the hot," and the candidate coefficient "0.74" is associated with a plurality of attributes including "female," "normal," "young," and "sensitive to the cold." In this case, if all of pieces of information indicating "male," "slender," "young," and "sensitive to the hot" are entered through the input unit (12), the candidate coefficient "0.78" is selected as the appropriate coefficient α. If all of pieces of information indicating "female," "normal," "young," and "sensitive to the cold" are entered through the input unit (12), the candidate coefficient "0.74" is selected as the appropriate coefficient α.

### -Third Example of Procedure of Selecting Appropriate Coefficient α-

In the third example, the input unit (12) is configured to receive input of the appropriate coefficient α. In this case, a user (e.g., a target) selects the appropriate coefficient α, and enters through the input unit (12) the appropriate coefficient α thus selected. As a result, the appropriate coefficient α is stored in the storage (13) (see FIG. 1).

The configuration of the third example can be effectively used by being combined with the configuration of the first example or the configuration of the second example. For example, if the target wishes to adjust the appropriate coefficient α after the controller (14C) selects the appropriate coefficient α using the configuration of the first example or the configuration of the second example, the target enters the regulated appropriate coefficient α through the input unit (12) using the configuration of the third example. Thus, the appropriate coefficient α selected by the controller (14C) can be adjusted to a value based on the target's sensation.

### -First Example of Procedure of Selecting Maximum Variance B-

A first example of a procedure of selecting the maximum variance B (see FIG. 1) will be described.

First, the blood flowmeter (20) measures the time series data on the target's blood flow within a predetermined time. The measurement by the blood flowmeter (20) is repeated plural times within the predetermined time. As a result of this, a plurality of pieces of time series data on the target's blood flow are acquired. At this time, it is preferable that the target be present in an environment where the target feels comfortable.

Next, the variances of the plurality of pieces of time series data on the blood flow are calculated out. The maximum variance B is selected as the maximum one of the plurality of variances calculated in this manner.

### -Second Example of Procedure of Selecting Maximum Variance B-

The configuration of the determination system (1) for selecting the maximum variance B (see FIG. 1) will be described with reference to FIG. 5.

As illustrated in FIG. 5, the determination system (1) further includes a body motion sensor (40) that detects a body motion of the target. The body motion sensor (40) includes, for example, a three-dimensional acceleration sensor. The body motion sensor (40) is fitted to the target, and detects a composite acceleration of the target generated by the target's motion, the composite acceleration being a composite of accelerations of the target's motion in the XYZ directions. The body motion sensor (40) is connected to the comfort determination device (10), and outputs information indicating a detection result of the body motion sensor (40) (a body motion signal) to the comfort determination device (10).

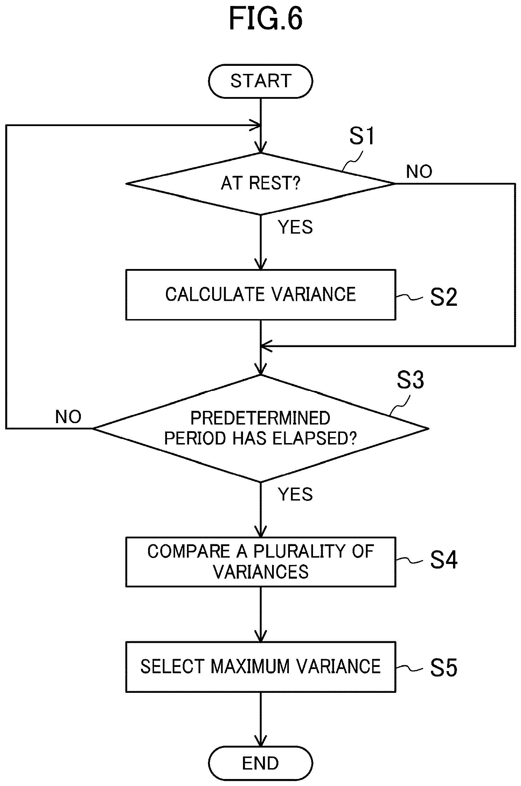

A second example of a procedure of selecting the maximum variance B will be described with reference to FIGS. 1, 5, and 6. FIG. 6 is a flowchart showing the second example of the procedure of selecting the maximum variance B.

As shown in FIGS. 1, 5, and 6, in step S1, the acquisition unit (11A) of the comfort determination device (10) acquires the body motion signal from the body motion sensor (40). The controller (14C) of the comfort determination device (10) determines whether or not the target is at rest, based on the body motion signal. For example, if the target's composite acceleration indicated by the body motion signal is equal to or smaller than 0.05G, the controller (14C) determines that the target is at rest. On the other hand, if the target's composite acceleration indicated by the body motion signal is greater than 0.05G, the controller (14C) determines that the target is not at rest.

If the controller (14C) determines that the target is at rest ("Yes" in step S1), the process proceeds to step S2. If the controller (14C) determines that the target is not at rest ("No" in step S1), the process proceeds to step S3.

In step S2, the blood flowmeter (20) measures time series data on the target's blood flow. The acquisition unit (11A) of the comfort determination device (10) acquires the time series data on the blood flow from the blood flowmeter (20), and the calculator (14A) calculates the variance of the time series data on the blood flow. In this way, the variance of the time series data on the target's blood flow obtained when a determination is made that the target is at rest is calculated out.

In step S3, the controller (14C) of the comfort determination device (10) determines whether or not a predetermined period has elapsed since the start of the process for selecting the maximum variance B. If the controller (14C) determines that the predetermined period has elapsed ("Yes" in step S3), the process proceeds to step S4. If the controller (14C) determines that the predetermined period has not elapsed ("No" in step S3), the process proceeds to step S1.

As a result of repeating the process shown in step S2 plural times within the predetermined period, a plurality of variances of time series data on the blood flow is calculated out.

In step S4, the controller (14C) of the comfort determination device (10) compares the plurality of variances of the time series data on the blood flow to each other.

In step S5, the controller (14C) of the comfort determination device (10) selects a maximum one of the plurality of variances of the time series data on the blood flow compared to each other in step S4, as the maximum variance B. As a result, the process ends.

### -Advantages of This Embodiment-

As described above with reference to FIGS. 1 to 6, the calculator (14A) calculates the index of fluctuation indicating fluctuations in the target's blood flow, and the determiner (14B) determines whether or not the target is comfortable, based on the index of fluctuation. The fluctuations in the blood flow have a correlation with determination results of the determination as to whether or not the target is comfortable. The fluctuations in the blood flow are less likely to be affected by factors irrelevant to the target's thermal sensation (e.g., a situation where the target sweats and a situation where the target is exposed to wind) than the skin temperature is. Thus, determining whether or not the target is comfortable, based on the index of fluctuation indicating fluctuations in the blood flow, can reduce accuracy deterioration of the determination result.

The calculator (14A) calculates the variance of the time series data on the blood flow as the index of fluctuation. This allows the determiner (14B) to determine whether or not the target is comfortable, with attention given to only the variance of the time series data on the blood flow. This can reduce the computational load on the determiner (14B).

The maximum variance B and the appropriate coefficient α merely need to be stored in the storage (13) to allow the determiner (14B) to determine whether or not the target is comfortable (see FIG. 1). This can reduce an increase in the capacity of the storage (13).

In addition, only sensing the target's blood flow with the blood flowmeter (20) allows the determiner (14B) to determine whether or not the target is comfortable. This can reduce time and effort that it takes for the user to operate the comfort determination device (10).

Changing the appropriate coefficient α enables regulation of the threshold α·B for determining whether or not the target is comfortable. Thus, the threshold α·B can be easily regulated.

### «Other Embodiments»

While the embodiment and variations thereof have been described above, it will be understood that various changes in form and details may be made without departing from the spirit and scope of the claims (e.g., (1) and (2)). The foregoing embodiment and variations thereof may be combined and replaced with each other without deteriorating the intended functions of the present disclosure.

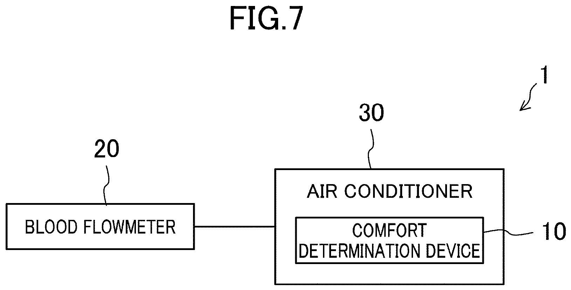

(1) As shown in FIG. 7, the determination system (1) may further include an air conditioner (30), which includes the comfort determination device (10). In this case, the comfort determination device (10) is made up of, for example, electronic components provided in a housing of the air conditioner (30). In the determination system (1), the comfort determination device (10) may be configured as an external device separate from the air conditioner (30), and the comfort determination device (10) and the air conditioner (30) may be communicably connected together in a wired or wireless manner. The air conditioner (30) cools or heats indoor air so that the air temperature in an indoor space reaches a set temperature.

The air conditioner (30) is communicably connected to the blood flowmeter (20). The air conditioner (30) that has acquired the time series data on the target's blood flow from the blood flowmeter (20) causes the comfort determination device (10) to determine whether or not the target is comfortable. The air conditioner (30) performs an appropriate process based on the determination result of the comfort determination device (10). The appropriate process includes, for example, a first process and a second process. The first process is a process in which if the comfort determination device (10) determines that the target is comfortable (see step S15 in FIG. 3), the set temperature of the air conditioner (30) is not changed. The second process is a process in which if the comfort determination device (10) determines that the target is not comfortable (see step S17 in FIG. 3), the set temperature of the air conditioner (30) is changed until the comfort determination device (10) determines that the target is comfortable.

In the air conditioner (30), the determiner (14B) of the comfort determination device (10) determines whether or not the target is comfortable. Thus, if a plurality of targets are present, the determiner (14B) of the comfort determination device (10) can determine whether or not the targets are comfortable, on a target-by-target basis. As a result, if any one of the plurality of targets is a user of the air conditioner (30), the comfort determination device (10) determines whether or not the user of the air conditioner (30) is comfortable. This allows the air conditioner (30) to perform temperature control adapted to how the user of the air conditioner (30) perceives the air temperature.

(2) In the air conditioner (30) shown in FIG. 7, the appropriate coefficient α (see FIG. 1) may be selected to be any one of a plurality of coefficients α1 to αm. That is to say, the appropriate coefficient α may be selected in many stages. The character m is an integer of two or more. The coefficients α1 to αm decrease in the order of the coefficients α1 to αm (1 > α1 > α2 > ... > αm > 0). The process for selecting the appropriate coefficient α to be any one of the plurality of coefficients α1 to αm is performed, for example, by the user entering an intended coefficient through a remote control (remote controller) for the air conditioner (30).

If, in the second process described in the section (1), a process is performed to change the set temperature of the air conditioner (30), the range of change in the set temperature may be varied in accordance with which one of the coefficients α1 to αm is selected as the appropriate coefficient α. For example, the lower the degree of comfort is (the smaller the one of the coefficients α1 to αm selected as the appropriate coefficient is), the greater the range of change in the set temperature of the air conditioner (30) is. The controller (14C) of the comfort determination device (10) (see FIG. 1), for example, performs a process to vary the range of change in the set temperature of the air conditioner (30) in accordance with the one of the coefficients α1 to αm selected as the appropriate coefficient α.

### INDUSTRIAL APPLICABILITY

As can be seen from the foregoing description, the present disclosure is useful for a comfort determination device, an air conditioner, and a comfort determination method.

### DESCRIPTION OF REFERENCE CHARACTERS

- 10: Comfort Determination Device
- 11: Acquisition Unit
- 12: Input Unit (First Input Unit, Second Input Unit)
- 14A: Calculator
- 14B: Determiner
- 14C: Controller
- 30: Air Conditioner
- 40: Body Motion Sensor

## Claims

1. A comfort determination device, comprising:
an acquisition unit (11A) configured to acquire time series data on a blood flow of a target;
a calculator (14A) configured to calculate an index of fluctuation indicating fluctuations in the blood flow of the target from the time series data on the blood flow acquired by the acquisition unit (11A); and
a determiner (14B) configured to determine whether or not the target is comfortable, based on the index of fluctuation calculated by the calculator (14B).

2. The comfort determination device of claim 1, wherein
the calculator (14A) calculates a variance of the time series data on the blood flow acquired by the acquisition unit (11A) as the index of fluctuation.

3. The comfort determination device of claim 2, wherein
the determiner (14B) selects a threshold for determining whether or not the target is comfortable based on a maximum one of such variances of the target thus calculated in the past.

4. The comfort determination device of claim 3, further comprising:
a first input unit (12) configured to receive input of information indicating whether or not the target has felt comfortable; and
a controller (14C) configured to select an appropriate coefficient based on a calculation result of the control device (14) and an input result received by the first input unit (12),
the threshold indicating a value obtainable by multiplying the maximum one of the variances by the appropriate coefficient.

5. The comfort determination device of claim 3, further comprising:
a second input unit (12) configured to receive input of information related to an attribute of the target; and
a controller (14C) configured to select an appropriate coefficient based on an input result entered into the second input unit (12),
the threshold indicating a value obtainable by multiplying the maximum variance by the appropriate coefficient.

6. The comfort determination device of any one of claims 3 to 5, wherein
the maximum one of the variances indicates a maximum value of the plurality of variances calculated within a predetermined period.

7. The comfort determination device of any one of claims 3 to 5, wherein
the maximum one of the variances indicates a maximum value of the plurality of variances calculated by the calculator (14A) while the target is determined as being at rest based on measurement results of a body motion sensor (40) measuring a state of the target.

8. An air conditioner comprising the comfort determination device (10) of any one of claims 1 to 7.

9. A comfort determination method, comprising:
acquiring time series data on a blood flow of a target;
calculating an index of fluctuation indicating fluctuations in the blood flow of the target from the time series data on the blood flow; and
determining whether or not the target is comfortable, based on the index of fluctuation.
